# EUROPÄISCHE PATENTSCHRIFT

(11) **EP 2 512 547 B1**
(45) Veröffentlichungstag und Bekanntmachung des Hinweises auf die Patenterteilung: **06.05.2015**
(21) Anmeldenummer: 10803227.7
(22) Anmeldetag: 15.12.2010
(51) Int. Cl.: A61M 1/16

(54) **BILANZIEREINRICHTUNG, EXTERNE MEDIZINISCHE FUNKTIONSEINRICHTUNG, BEHANDLUNGSVORRICHTUNG**
BALANCING DEVICE, EXTERNAL MEDICAL FUNCTIONAL DEVICE, TREATMENT DEVICE
DISPOSITIF D'ÉQUILIBRAGE, DISPOSITIF FONCTIONNEL MÉDICAL EXTERNE, DISPOSITIF DE TRAITEMENT

(30) Priorität: 16.12.2009 DE 102009058681
(43) Veröffentlichungstag der Anmeldung: 24.10.2012
(73) Patentinhaber: Fresenius Medical Care Deutschland GmbH, 61352 Bad Homburg (DE)
(72) Erfinder: PETERS, Arne, 61352 Bad Homburg (DE); HEIDE, Alexander, 65817 Eppstein (DE)
(74) Vertreter: Bobbert & Partner Patentanwälte PartmbB
(86) Internationale Anmeldenummer: PCT/EP2010/007647
(87) Internationale Veröffentlichungsnummer: WO 2011/082783

(56) Entgegenhaltungen:
- EP-A1- 0 623 357
- EP-A1- 0 900 572
- US-A- 3 709 222
- US-A- 4 997 570
- US-A1- 2009 095 679

## Beschreibung

Die vorliegende Erfindung betrifft eine Bilanziereinrichtung gemäß Anspruch 1. Sie betrifft ferner eine externe medizinische Funktionseinrichtung gemäß Anspruch 7 sowie eine Behandlungsvorrichtung gemäß Anspruch 10.

Aus der EP 0 867 195 B1 sind Bilanziereinrichtungen zum Bilanzieren von Mengen- und/oder Volumenströmen medizinischer Fluide wie Blut oder während der Blutbehandlung eingesetzter Fluide bekannt.

Die US 4,997,570 A offenbart eine Bilanziereinrichtung mit einer Pumpe.

Die EP 0 623 357 A1 beschreibt eine Dialysiereinrichtung mit Pumpen.

Die US 3,709,222 A beschreibt eine Dialysiereinrichtung mit rotierenden Peristaltikpumpen.

Die EP 0 900 572 A1 offenbart eine Zentrifugalpumpe zum Fördern eines Fluids.

Die US 2009/095679 A1 offenbart pneumatisch angetriebene Dialysatverdrängerpumpen.

Eine Aufgabe der vorliegenden Erfindung ist es, eine weitere Bilanziereinrichtung vorzuschlagen.

Diese Aufgabe wird durch eine Bilanziereinrichtung zum Bilanzieren medizinischer Fluide, insbesondere zum Bilanzieren von Dialysat, mit den Merkmalen des Anspruchs 1 gelöst.

Die erfindungsgemäße Bilanziereinrichtung weist wenigstens eine Bilanzierkammer und wenigstens eine Fördereinrichtung zum Befüllen der Bilanzierkammer auf.

Vorteilhafte Weiterbildungen sind Gegenstand der Unteransprüche und Ausführungsformen.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen.

Unter dem Begriff "Bilanzierung", wie er hierin verwendet wird, ist in einer Ausführungsform eine Gegenüberstellung von Mengen und/oder Volumina medizinischer Fluide zu verstehen, die einem Patienten oder einer Behandlungsvorrichtung zum Behandeln des Patienten zu- oder abgeführt werden.

Der Begriff "Patient", wie er hierin verwendet wird, bezeichnet einen Menschen oder ein Tier, gleich ob krank oder gesund.

Eine "Bilanzierkammer" im Sinne der vorliegenden Erfindung bezeichnet eine Einrichtung, welche dazu vorgesehen ist, die zur Bilanzierung vorgesehenen medizinischen Fluide - oder Teilmengen derselben - in einem Inneren bzw. Innenvolumen aufzunehmen.

Die Bilanzierkammer ist in einer erfindungsgemäßen Ausführungsform eine Kammer, die durch wenigstens eine Trennwand oder Membran, welche verschiebbar oder flexibel ausgestaltet sein kann, in wenigstens zwei Bilanzierkammerhälften oder -abschnitte unterteilt ist. Wenigstens eine der Bilanzierkammerhälften oder-abschnitte kann vorgesehen sein, zugeführte bzw. frische medizinische Fluide aufzunehmen. Wenigstens eine weitere Bilanzierkammerhälfte oder ein weiterer

Bilanzierkammerabschnitt kann vorgesehen sein, abgeführte bzw. verbrauchte medizinische Fluide aufzunehmen.

Die Bilanziereinrichtung kann mehr als eine Bilanzierkammer, d.h. z.B. zwei, drei, vier oder mehr Bilanzierkammern, aufweisen.

Mehrere, z.B. zwei, Bilanzierkammern können beispielsweise vorteilhaft dazu eingesetzt werden, einen kontinuierlichen Fluss der medizinischen Fluide während der Bilanzierung sicherzustellen.

Die Bilanzierkammern können miteinander in Fluidverbindung stehen oder nicht. Die Bilanzierkammern können gemeinsam oder getrennt voneinander befüll-und/oder entleerbar sein.

Jede Bilanzierkammer kann (eine oder mehrere) Zuführleitungen für zugeführte oder frische medizinische Fluide und (eine oder mehrere) mit einem Auslass verbundene Abführleitungen für abgeleitete verbrauchte medizinische Fluide aufweisen. In den Zuführ- und/oder in den Abführleitungen können Absperrventile angeordnet sein.

Beispiele für solche Absperrventile schließen Aktuatoren, welche aus einem Abschnitt einer Maschine, wie einer Behandlungsvorrichtung, heraus- und/oder hereinfahrbar sind, ein. Mittels dieser Aktuatoren kann es möglich sein, einen Fluidstrom innerhalb eines Fluidsystems der medizinischen Fluide zu unterbinden oder freizugeben. Derartige Aktuatoren schließen die in der von der Anmelderin der vorliegenden Anmeldung am 10. Juni 2009 beim Deutschen Patent- und Markenamt hinterlegten Anmeldung 10 2009 024 468.9 mit dem Titel *"Externe Funktionseinrichtung,*

*Blatbehandlungsvorrichtung zum Aufnehmen einer erfindungsgemäßen externen Funktionseinrichtung, sowie Verfahren"* als "Phantomventile" bezeichneten Aktuatoren ein.

Beispiele für Bilanzierkammern gemäß einer Ausführungsform der Bilanziereinrichtung der vorliegenden Erfindung sowie deren jeweilige Funktion sind in der eingangs genannten EP 0 867 195 B1 der Anmelderin der vorliegenden Anmeldung offenbart.

Die Fördereinrichtung kann Teil eines Fluidsystems sein, in welchem das medizinische Fluid vorliegt bzw. enthalten ist. Die Fördereinrichtung kann z.B. zum Fördern des medizinischen Fluids in das Fluidsystem eingebaut bzw. geschaltet sein. Die Fördereinrichtung kann in ihrem Gebrauch von dem zu bilanzierenden medizinischen Fluid durchströmt sein.

Das Fluidsystem kann Leitungen, Schläuche, Schlauchsysteme, Kanäle, Kammern, Ausbuchtungen, Einrichtungen oder Räume oder Bereiche zum Speichern oder Vorhalten oder Zurückhalten von Fluiden sowie Steuer- oder Regeleinrichtungen zum Steuern oder Regeln eines Strömungsdurchflusses der Fluide und dergleichen aufweisen.

Das Fluidsystem ist in bestimmten Ausführungsformen für eine Dialysierflüssigkeit vorgesehen.

Das Fluidsystem ist in bestimmten Ausführungsformen für Blut in einem extrakorporalen Blutkreislauf oder andere Fluide vorgesehen. Die anderen Fluide umfassen eine Citrat- und/oder Calciumlösung, Wasser oder eine Hydraulikflüssigkeit.

Der in der Bilanzierkammer beim Befüllen herrschende Druck wird im Folgenden als Fülldruck bezeichnet. Er kann veränderbar sein. Er kann ansteigend sein. Er kann zu verschiedenen Momenten unterschiedlich groß sein.

In einer Ausführungsform ist ein maximaler Fülldruck der erfindungsgemäßen Bilanziereinrichtung über eine eingestellte Drehzahl der Fördereinrichtung festlegbar. Der maximale Fülldruck kann durch die Kennlinie der Fördereinrichtung, z.B. eine Pumpenkennlinie, vorgegeben sein.

In einer Ausführungsform wird der Fluss und/oder der Förderdruck der Fördereinrichtung durch geeignete Mittel gemessen. Entsprechende Mittel können dazu konfiguriert und vorgesehen sein.

Der maximale Fülldruck zum Befüllen der Bilanzierkammer der Bilanziereinrichtung kann vorgegeben sein. Der maximale Fülldruck ist in einer erfindungsgemäßen Ausführungsform durch Änderung der Betriebsparameter der Fördereinrichtung (z. B. mittels einer Beeinflussung der Drehzahl einer Pumpe) einstellbar bzw. wird eingestellt.

In einer weiteren erfindungsgemäßen Ausführungsform sind die Betriebsparameter der Fördereinrichtung (z. B. mittels einer Beeinflussung der Drehzahl einer Pumpe) durch eine Änderung eines magnetischen Feldes einstellbar bzw. wird eingestellt.

Der maximale Fülldruck kann in einer Ausführungsform nach seinem Erreichen von der Fördereinrichtung für eine Zeit konstant gehalten werden oder, in einer anderen Ausführungsform, abfallen. Dies kann in Abhängigkeit der Vorlast geschehen.

Der maximale Fülldruck kann beispielsweise erreicht sein, wenn die Bilanzierkammer im Wesentlichen oder vollständig mit dem oder den zu bilanzierenden medizinischen Fluiden gefüllt oder befüllt ist.

Der maximale Fülldruck kann auch erreicht sein, wenn eine Bilanzierkammerhälfte oder ein Bilanzierkammerabschnitt durch Betreiben der Fördereinrichtung im Wesentlichen oder vollständig befüllt wurde.

Die "druckbegrenzte Fördereinrichtung" ist in einer Ausführungsform der erfindungsgemäßen Bilanziereinrichtung eine Fördereinrichtung, welche nach Erreichen eines maximalen Drucks oder Fülldrucks keinen höheren Druck im Inneren der Bilanzierkammer mehr aufbaut.

Die "druckbegrenzte Fördereinrichtung" ist in einer anderen Ausführungsform der erfindungsgemäßen Bilanziereinrichtung eine Pumpe, deren Laufrad bei Erreichen des maximalen Fülldrucks vom geförderten Fluid überströmt wird.

Die "druckbegrenzte Fördereinrichtung" ist in einer weiteren Ausführungsform der erfindungsgemäßen Bilanziereinrichtung eine Fördereinrichtung, welche in wenigstens einem Betriebszustand als Konstantdruckquelle oder als Druckquelle mit konstantem oder annähernd konstantem Druck betrieben werden kann.

Die "druckbegrenzte Fördereinrichtung" ist in einer weiteren Ausführungsform der erfindungsgemäßen Bilanziereinrichtung eine Fördereinrichtung, welche wenigstens eine Pumpe aufweist oder aus einer solchen besteht, wobei die Pumpe keine Überströmventile und/oder Bypassleitungen aufweist oder mit solchen funktionell verbunden ist.

Die "druckbegrenzte Fördereinrichtung" ist in einer weiteren Ausführungsform der erfindungsgemäßen Bilanziereinrichtung eine Fördereinrichtung, welche nicht mit einer Steuereinrichtung zu dem Zweck verbunden ist - und/oder keine zu diesem Zweck vorgesehene und entsprechende konfigurierte Steuereinrichtung aufweist -, eine Druckbegrenzung der Fördereinrichtung in Abhängigkeit mit einem während der Befüllung der Bilanzierkammer des in dieser herrschenden Fülldrucks vorzunehmen.

Die "druckbegrenzte Fördereinrichtung" ist in der erfindungsgemäßen Bilanziereinrichtung eine Pumpe, welche aufgrund ihrer Bauart keinen Druck über einen vorbestimmten Druck hinaus - hier den Fülldruck - aufbaut. Sie wird hierin in wenigstens einer Ausführungsform der erfindungsgemäßen Bilanziereinrichtung durch keine weiteren Elemente, insbesondere keine Steuerung, Schaltung, Ventile, Bypassüberdruckventile, Druckmesseinrichtungen und dergleichen, jeweils direkt oder indirekt, unterstützt.

Die "druckbegrenzte Fördereinrichtung" ist in einer Ausführungsform der erfindungsgemäßen Bilanziereinrichtung eine Fördereinrichtung, bei deren Einsatz nach Erreichen eines maschinenseitig eingestellten maximalen Drucks oder Fülldrucks die von ihr befüllte Bilanzierkammer als volumenstarre Kammer angenommen werden kann.

Die Bilanziereinrichtung weist in einer erfindungsgemäßen Ausführungsform mehrere Fördereinrichtungen auf.

Die Bilanziereinrichtung weist in einer erfindungsgemäßen Ausführungsform keine Überströmventile, Bypassleitungen, Steuerungen, Schaltungen, Ventile, Bypassüberdruckventile, Druckmesseinrichtungen und dergleichen auf, welche zum Begrenzen des Förderdrucks der Fördereinrichtung geeignet und vorgesehen oder konfiguriert sind.

Die Bilanziereinrichtung weist in einer erfindungsgemäßen Ausführungsform keine Rollenpumpe oder Zahnradpumpe mit Bypassventil und/oder Druckregelung auf.

Weist die erfindungsgemäße Bilanziereinrichtung mehrere Fördereinrichtungen auf, so können diese in einer Ausführungsform gleichartig oder unterschiedlich ausgestaltet sein.

In einer erfindungsgemäßen Ausführungsform der Bilanziereinrichtung weist diese mehrere, in Reihe geschaltete Fördereinrichtungen auf. Auf diese Weise kann es vorteilhaft möglich sein, die Bilanzierkammer gezielt zu entlasten.

Die einzelnen Fördereinrichtungen sind in einer erfindungsgemäßen Ausführungsform der Bilanziereinrichtung angeordnet, um in die gleiche Förderrichtung zu laufen bzw. betrieben zu werden bzw. um in der gleichen Richtung fördern.

In einer erfindungsgemäßen Ausführungsform laufen die Fördereinrichtungen in verschiedenen Richtungen bzw. fördern in entgegengesetzte Richtungen. Hierbei kann es vorteilhaft möglich sein, gezielt Druck aufzubauen und/oder den Volumenstrom der medizinischen Fluide zu begrenzen. Dies kann vorteilhaft dazu beitragen, die auf die Bilanzierkammmer wirkenden Kräfte weiter zu verringern. Insbesondere kann es vorteilhaft möglich sein, die auf die Bilanzierkammer und deren Wände wirkenden Kräfte zu verringern oder gar zu minimieren.

In einer erfindungsgemäßen Ausführungsform laufen wenigstens zwei Fördereinrichtungen in derselben Richtung bzw. fördern in derselben Richtung. Hierbei kann es vorteilhaft möglich sein, gezielt Druck abzubauen. Auch dies kann vorteilhaft dazu beitragen, die auf die Bilanzierkammer wirkenden Kräfte weiter zu verringern (Entlastung der Kammer). Insbesondere kann es vorteilhaft möglich sein, die auf die Bilanzierkammer und deren Wände wirkenden Kräfte zu verringern oder gar zu minimieren.

Eine "Zentrifugalpumpe" oder eine Kreiselpumpe kann in einer Ausführungsform vorteilhaft einen hohen Volumenstrom bei niedrigen Drücken und/oder einen niedrigen Volumenstrom bei hohen Drücken bereitstellen.

Die Zentrifugalpumpe ist in einer erfindungsgemäßen Ausführungsform eine Axialpumpe mit den dem Fachmann im Zusammenhang mit Axialpumpen bekannten Vorteilen.

Die Zentrifugalpumpe ist in einer weiteren erfindungsgemäßen Ausführungsform eine Radialpumpe oder eine Diagonalpumpe mit den dem Fachmann im Zusammenhang mit Radialpumpen bzw. Diagonalpumpen bekannten Vorteilen.

Der maximale Druck der Zentrifugalpumpe - und damit der maximale Fülldruck - kann über die Drehzahl, z.B. mittels Drehzahlbegrenzung, eingestellt werden, so dass die maximale Druckbelastung auf das gesamte System vorteilhaft (sehr) exakt definierbar sein kann.

Die Zentrifugalpumpe kann die Eigenschaft aufweisen, dass ab einem gewissen Fluiddruck, z.B. wenn die Bilanzierkammer vollständig gefüllt ist, eine Überströmung des Laufrads bzw. des Rotationsabschnitts auftritt. Diese Überströmung kann eine Druckbegrenzung im geförderten Fluid zur Folge haben, derart, dass die Zentrifugalpumpe druckbegrenzt im Sinne der vorliegenden Erfindung arbeitet.

Zum Erzielen der Druckbegrenzung bedarf die Zentrifugalpumpe in einer Ausführungsform vorteilhafter Weise keiner Unterstützung durch weitere Bauteile wie Steuerung, Regelung, Ventile, usw.

Unter einer Druckquelle wird erfindungsgemäß jegliche Fluidförderungsvorrichtung verstanden, deren Ausgangsfluiddruck konstant oder im Wesentlichen konstant ist.

In einer weiteren bevorzugten Ausführungsform weist die Fördereinrichtung wenigstens einen drehenden bzw. rotierenden Abschnitt bzw. einen Rotationsabschnitt auf. Dieser ist in einer erfindungsgemäßen Ausführungsform mechanisch gelagert, in einer anderen ist er magnetisch gelagert.

Der Rotationsabschnitt kann ausschließlich oder ergänzend magnetisch gelagert sein.

Der Rotationsabschnitt kann in einem Inneren der Fördereinrichtung angeordnet sein.

Der Rotationsabschnitt kann im Gebrauch vollständig von den durch die Fördereinrichtung strömenden medizinischen Fluiden umspült sein.

Der Rotationsabschnitt ist in einer Ausführungsform ein Laufrad oder ein Rotor.

In einer weiteren Ausführungsform weist die Fördereinrichtung wenigstens einen Rotationsabschnitt auf, welcher vorgesehen und ausgestaltet ist, um mittels eines externen Antriebs magnetisch oder mittels eines elektrischen Feldes antreibbar zu sein oder betrieben zu werden.

In einer weiteren Ausführungsform ist der externe Antrieb des Rotationsabschnitts ausgestaltet, um mechanisch, z.B. über lösbare, fluiddichte Kupplungen angetrieben zu werden.

Der Begriff "externer Antrieb", wie er hierin verwendet wird, bezeichnet einen Antrieb für den Rotationsabschnitt, welcher Teil der Bilanziereinrichtung sein kann, nicht aber Teil sein muss.

Der externe Antrieb kann an einer Vorrichtung, welche mit einer erfindungsgemäßen Bilanziereinrichtung zusammenwirkt, wie einer Behandlungsvorrichtung, angeordnet sein. Der externe Antrieb kann Teil der Vorrichtung sein.

Die magnetische Antriebskraft oder -wirkung kann mit Hilfe eines oder mehrerer Magneten erzielt werden. Sie kann mit Hilfe von stromdurchflossenen Leitern erzielt werden. Beispielsweise können stromdurchflossene Spulen verwendet werden.

Die Fördereinrichtung ist in einer erfindungsgemäßen Ausführungsform der Bilanziereinrichtung eine magnetisch gelagerte Zentrifugalpumpe, wie sie beispielsweise in der EP 0 900 572 A1 offenbart ist.

Eine solche magnetisch gelagerte Zentrifugalpumpe kann wie jede andere magnetisch gelagerte Fördereinrichtung im Sinne der vorliegenden Erfindung den Vorteil bieten, dass keine mechanische und/oder elektrische Schnittstelle zur Maschine erforderlich ist und/oder keine Fluide von der Maschine bzw. der Behandlungsvorrichtung zur Pumpe übertragen werden müssen.

In einer weiteren bevorzugten Ausführungsform ist das medizinische Fluid ausgewählt aus Dialysierflüssigkeit, Blut, Substituatflüssigkeit, Medikamenten, Medikamentenzubereitungen sowie Mischungen oder Kombinationen derselben.

Insbesondere ist eine Bilanzierung in einer erfindungsgemäßen Ausführungsform auf der Dialysatseite während einer Dialyse erfindungsgemäß angedacht.

In einer erfindungsgemäßen Ausführungsform ist eine Bilanzierung auf der Blutseite während einer Dialyse erfindungsgemäß angedacht.

Weitere Fluide, die zu einer Bilanzierung im Rahmen einer Blutbehandlung eines Patienten von Interesse und/oder erforderlich sein können, schließen Lösungen bzw. in gelöster Form vorliegende Stoffwechselprodukte des Patienten, wie beispielsweise harnpflichtige Substanzen, und dergleichen ein.

In einer weiteren bevorzugten Ausführungsform ist wenigstens eine erste Fördereinrichtung vorgesehen, um in einer ersten Richtung zu fördern. Es ist ferner wenigstens eine zweite Fördereinrichtung vorgesehen, um in einer zweiten, der ersten entgegengesetzten Richtung, zu fördern.

Die Aufgabe der vorliegenden Erfindung wird ferner durch eine externe medizinische Funktionseinrichtung gemäß Anspruch 7 gelöst. Alle mit der erfindungsgemäßen Bilanziereinrichtung erzielbaren Vorteile lassen sich in bestimmten Ausführungsformen ungeschmälert auch mit der erfindungsgemäßen externen medizinischen Funktionseinrichtung erzielen, welche wenigstens eine erfindungsgemäße Bilanziereinrichtung aufweist.

In einer Ausführungsform der vorliegenden Erfindung ist die externe medizinische Funktionseinrichtung als externer Flüssigkeitskreislauf mit Dialysat- und extrakorporalem Blutkreislauf, oder ist als Blut- bzw. Dialysatkassette oder kombinierte Blut-/Dialysatkassette ausgestaltet. Die externe medizinische Funktionseinrichtung kann z.B. eine Blut- bzw. Dialysatkassette oder eine kombinierte Blut-/Dialysatkassette für die Dialyse sein.

In einer erfindungsgemäßen Ausführungsform ist die externe medizinische Funktionseinrichtung ein Disposable, ein Einmalartikel oder ein Ein-Weg-Produkt.

In einer erfindungsgemäßen Ausführungsform ist die externe medizinische Funktionseinrichtung eine Disposable-Kassette.

Die Disposable-Kassette kann ein Hartteil sein. Sie kann aus einem Kunststoffmaterial hergestellt sein. Die Disposable-Kassette kann mittels eines Spritzgussverfahrens hergestellt sein.

Die Aufgabe der vorliegenden Erfindung wird ferner durch eine Behandlungsvorrichtung gemäß Anspruch 10 gelöst. Alle mit der erfindungsgemäßen Bilanziereinrichtung erzielbaren Vorteile können in bestimmten Ausführungsformen ungeschmälert auch mit der erfindungsgemäßen Behandlungsvorrichtung erzielt werden.

Die erfindungsgemäße Behandlungsvorrichtung ist zum Behandeln medizinischer Fluide geeignet. Sie ist dazu ausgestaltet, wenigstens eine erfindungsgemäße Bilanziereinrichtung zu betreiben.

Zumindest zu diesem Zweck kann die Behandlungsvorrichtung eine Steuer- oder Regeleinrichtung aufweisen. Die Steuer- oder Regeleinrichtung kann ein Mikroprozessor sein oder einen solchen aufweisen.

In einer bevorzugten Ausführungsform der erfindungsgemäßen Behandlungsvorrichtung weist die Behandlungsvorrichtung eine Einrichtung auf, die dazu vorgesehen und konfiguriert ist, die Fördereinrichtung der Bilanziereinrichtung über eine magnetische Antriebsschnittstelle anzutreiben.

Die Einrichtung kann beispielsweise ein Magnet oder ein magnetisch wirkendes System und/oder ein stromdurchflossener Leiter, wie beispielsweise eine oder mehrere stromdurchflossene Spulen, sein oder diese aufweisen.

Die Behandlungsvorrichtung kann funktionell mit einer erfindungsgemäßen Bilanziereinrichtung und/oder mit einer erfindungsgemäßen externen medizinischen Funktionseinrichtung verbunden sein.

Die erfindungsgemäße Behandlungsvorrichtung weist in einer erfindungsgemäßen Ausführungsform wenigstens eine erfindungsgemäße Bilanziereinrichtung auf.

Die erfindungsgemäße Behandlungsvorrichtung ist in einer erfindungsgemäßen Ausführungsform mit der erfindungsgemäßen Bilanziereinrichtung fest verbunden.

Eine wiederholte Benutzung der fest verbundenen erfindungsgemäßen Bilanziereinrichtung ist in einer erfindungsgemäßen Ausführungsform vorgesehen.

Die erfindungsgemäße Behandlungsvorrichtung ist in einer erfindungsgemäßen Ausführungsform eine Hämodialyseeinrichtung.

Die Behandlungsvorrichtung weist in bestimmten Ausführungsformen ferner weitere Einrichtungen auf oder ist zur Koppelung mit diesen vorgesehen. Zu ihnen zählen beispielsweise ein extrakorporaler Blutkreislauf, Steuer- oder Regeleinrichtungen zum Ansteuern der Durchführung einer medizinischen Behandlung, Einrichtungen zum Überwachen und/oder Anzeigen einer Bilanzierung der während einer medizinischen Behandlung eingesetzten und/oder zirkulierten medizinischen Fluide, Einrichtungen zum Anzeigen oder Darstellen von Zuständen und/oder Parametern der medizinischen Behandlung oder der Bilanzierung, wie Bildschirmen und dergleichen, Einrichtungen zum Bedienen bzw. Betätigen oder Ansteuern einer oder mehrerer Komponenten der Behandlungsvorrichtung, wie Tastaturen und dergleichen, um z.B. die Durchführung einer medizinischen Behandlung zu veranlassen, und dergleichen.

In einer erfindungsgemäßen Ausführungsform ist die Behandlungsvorrichtung eine Blutbehandlungsvorrichtung.

Beispiele für Blutbehandlungsverfahren schließen Dialyseverfahren, wie eine Hämodialyse, insbesondere mittels Ultrafiltration, eine Hämodiafiltration, eine Peritonealdialyse, eine automatische Peritonealdialyse, und dergleichen ein. Die Blutbehandlungseinrichtung kann zum Durchführen dieser Verfahren entsprechend ausgestaltet sein.

Die erfindungsgemäße Bilanziereinrichtung kann nicht zuletzt bei einer Peritonealdialyse vorteilhaft zur Volumenbestimmung der Dialysierflüssigkeit, die in den Peritonealbereich des Patienten geleitet und/oder von dort aus dem Patienten befördert wird, eingesetzt werden. Dabei können zum Beispiel beide Bilanzierkammerhälften einer zweigeteilten Bilanzierkammer der Bilanziereinrichtung wechselseitig mit frischer Dialysierflüssigkeit (beim Einlaufen der Dialysierflüssigkeit in den Bauchraum des Patienten) und/oder mit gebrauchter Dialysierflüssigkeit (beim Entfernen der Dialysierflüssigkeit aus dem Bauchraum des Patienten) gefüllt werden. Die bei einer Bilanzierung interessierenden Volumina und/oder Mengen der medizinischen Fluide, z.B. der Dialysierflüssigkeit, können dabei beispielsweise über die Anzahl der Befüllungen der Bilanzierkammer bestimmt werden.

Ein Verfahren umfasst das Bilanzieren wenigstens eines medizinischen Fluids unter Verwenden wenigstens einer erfindungsgemäßen Bilanziereinrichtung oder wenigstens einer erfindungsgemäßen externen medizinischen Funktionseinrichtung oder wenigstens einer erfindungsgemäßen Behandlungsvorrichtung.

Ein Verfahren umfasst das Befüllen einer Bilanzierkammer mittels wenigstens einer Fördereinrichtung und das Betreiben der Fördereinrichtung in wenigstens einem Betriebszustand als Konstantdruckquelle.

Um die Fördereinrichtung in dem gewünschten Betriebszustand als Konstantdruckquelle zu betreiben, kann eine bestimmte Drehzahl der Fördereinrichtung eingestellt werden, bei der sich eine fixe bzw. feste oder vorgegebene Druckdifferenz über der Fördereinrichtung einstellen kann.

Die vorliegende Erfindung schlägt eine Bilanziereinrichtung vor, in welcher die Fördereinrichtung nach Befüllen der Bilanzierkammer(n) als Konstantdruckquelle betrieben wird.

Die Konstantdruckquelle kann vorteilhaft dazu beitragen, einen maximalen Fülldruck innerhalb der Bilanzierkammer sicherzustellen. Die baulichen Anforderungen an die Bilanzierkammer können damit geringer sein.

Allgemein kann eine Bilanziergenauigkeit vor allem von den Druckschwankungen zwischen zwei Füllvorgängen abhängen, was sich damit erklären lässt, dass der Schaltvorgang zum Beenden eines Befüllvorgangs geringfügigen Schwankungen unterliegt, und dass der Anstieg des Fülldrucks oder des Kammerinnendrucks gegen Ende der Befüllung erheblich zunimmt.

Ferner ist bekannt, dass eine Bilanzierkammer i.a.R. nicht druckstabil ist. Aus diesem Grund kann sich das Füllvolumen verändern.

Da die mittels der Fördereinrichtung in die Bilanzierkammer eingebrachten medizinischen Fluide in gleichem Maße bzw. mit gleicher Geschwindigkeit oder Rate die in der Bilanzierkammer vorhandenen Fluide verdrängen können, kann es vorteilhaft möglich sein, einen konstanten oder gleichmäßigen Mengen- und/oder Volumenstrom der zu bilanzierenden Fluide zu erreichen.

Da über die Drehzahl und/oder den maximalen Förderdruck der Fördereinrichtung auf vorteilhaft einfache Weise eine Druckdifferenz zum Betreiben der Bilanzierkammer eingestellt werden kann, kann die druckbegrenzte Fördereinrichtung einen vorteilhaft genau regelbaren (auch dynamisch regelbaren) Bilanzierkammerdruck während der Bilanzierung der medizinischen Fluide vorgeben.

Ein nachteiliger Druckanstieg und/oder Druckschwankungen der Bilanzierkammer können so vorteilhaft vermieden werden. Eine unerwünschte Volumenausdehnung oder -veränderung der Bilanzierkammer kann so vorteilhaft verhindert werden.

Auf diese Weise kann es vorteilhaft möglich sein, eine Mengen- und/oder Volumengenauigkeit einer Bilanzierung zu verbessern.

Dies kann zum Beispiel auch vorteilhaft dazu beitragen, das Fluidvolumen, das einem Patienten während einer Behandlung, z.B. einer Ultrafiltration während einer Dialysebehandlung, über die Dialysefiltermembran entzogen wird, genau zu bestimmen und/oder auf die vom behandelnden Arzt gewünschte Rate einzustellen. Die Sicherheit und ggf. auch die Verträglichkeit einer Blutbehandlung kann so vorteilhaft weiter verbessert werden.

So kann es vorteilhaft möglich sein, eine Fehlbilanzierung zu verhindern, wobei sich eine Fehlbilanzierung z.B. über die Dauer einer Blutbehandlungssitzung aufsummieren kann. Hat die Bilanzierung Auswirkung auf die durchgeführte Behandlung, so kann die mittels der erfindungsgemäßen Bilanziereinrichtung, in wenigstens einer Ausführungsform, verbesserte Bilanziergenauigkeit vorteilhaft zu einer verbesserten und/oder sichereren Behandlung - z.B. durch ein angemesseneres Einstellen einer Ultrafiltrationsrate - führen.

Die Bilanzierkammer der erfindungsgemäßen Bilanziereinrichtung kann vorteilhaft als druckbegrenzte volumetrische Bilanzierkammer mit hinreichender Festigkeit eingesetzt werden.

Auf aufwendige Konstruktionen, wie Verstrebungen oder verstärkte Kunststoffmaterialien oder dergleichen, mit welchen im Stand der Technik eine hinreichende Festigkeit gewährleistet werden müssen, kann bei Verwenden der erfindungsgemäßen Bilanziereinrichtung vorteilhaft verzichtet werden. Die Konstruktion der erfindungsgemäßen Bilanziereinrichtung kann somit aufgrund der vorgesehenen Druckbegrenzung mittels der Fördereinrichtung vorteilhaft vereinfacht werden.

Eine Abstützung von Wänden der Bilanzkammer an festen Strukturen der Behandlungsvorrichtung ist nicht erforderlich. Die Einsatzmöglichkeiten der Bilanziereinrichtung sind damit breiter geworden ohne Einbuße ihrer Funktionsgenauigkeit.

Daneben kann die Fördereinrichtung in der erfindungsgemäßen Bilanziereinrichtung vorteilhaft ohne die Verwendung von Sensoren und/oder Überströmventilen und/oder Bypassüberdruckventilen, insbesondere zum Zwecke der Druckbegrenzung in einem Inneren der Bilanzierkammer, und dergleichen auskommen. So kann es ferner vorteilhaft möglich sein, den Betrieb der Bilanziereinrichtung zu vereinfachen. Die Fördervorrichtung kann einfacher ausgestaltet sein.

Auf diese Weise können Abmessungen der Bilanziereinrichtung bzw. der Platzbedarf für die Bilanziereinrichtung vorteilhaft gering gehalten werden.

Durch eine magnetische Lagerung der Fördereinrichtung kann die Konstruktion der Fördereinrichtung vorteilhaft vereinfacht werden. So kann es vorteilhaft möglich sein, auf mechanische Bauteile, wie Lager und dergleichen, zu verzichten und auf diese Weise vorteilhaft einen geringen Verschleiß der Bauteile und/oder einen geringen Partikelabrieb sicherzustellen. Hierdurch bedingt kann vorteilhaft auch eine Erwärmung der Fördereinrichtung oder der Bilanziereinrichtung vermieden oder verringert werden.

Ferner kann die Fördereinrichtung der erfindungsgemäßen Bilanziereinrichtung vorteilhaft eine geringe Kavitationsneigung aufweisen.

Ein weiterer Vorteil kann eine nur geringe Geräuschentwicklung beim Gebrauch der erfindungsgemäßen Bilanziervorrichtung sein.

Da der Druck der Fördereinrichtung selbst bei anhaltendem Volumenstrom nach Abschluss der Befüllung der Bilanzierkammer nicht ansteigt, kann vorteilhaft umgangen werden, die Fördereinrichtung bei voller, d.h. im Wesentlichen oder vollständig befüllter, Bilanzierkammer abschalten zu müssen. So kann ein z.B. die Zentrifugalpumpe durchströmendes Fluid einen Rotationsabschnitt der Zentrifugalpumpe überströmen. Auf diese Weise kann eine gute Spülbarkeit (und Umspülbarkeit) der Fördereinrichtung sichergestellt werden mit einer gerichteten Strömung im durchströmten Raum der Zentrifugalpumpe.

Die magnetische Antriebsschnittstelle zum Betreiben der Fördereinrichtung bzw. eines Rotationsabschnitts derselben kann vorteilhaft einen berührungslosen und/oder dichtungsfreien Antrieb der Fördereinrichtung bereitstellen. Auf diese Weise kann es vorteilhaft möglich sein, auf offene Schnittstellen zwischen der Bilanziereinrichtung und der Behandlungsvorrichtung zu verzichten.

Somit kann es vorteilhaft möglich sein, einen besonders sicheren Betrieb der Bilanziereinrichtung sicherzustellen. Eine - wenngleich nur äußerst geringe - Kontaminationsgefahr der medizinischen Fluide kann somit vorteilhaft verringert und sogar ganz ausgeschlossen werden.

Die erfindungsgemäße Bilanziereinrichtung kann vorteilhaft als Disposable, d.h. als Wegwerfartikel für den Einmalgebrauch, eingesetzt werden. Da die Fördereinrichtung als integraler Bestandteil des Disposables vorgesehen sein kann, kann sie zusammen mit dem Disposable verworfen werden, wodurch die Sicherheit und Hygiene einer medizinischen Behandlung vorteilhaft weiter verbessert werden kann.

Die Verwendung von Zentrifugalpumpen hat in bestimmten Ausführungsformen gegenüber den druckgeregelten Schlauchrollen-, Zahnrad- oder Peristaltikpumpen den Vorteil der inhärenten Druckbegrenzung bei Okklusion flussabwärts der Pumpe. Der sich dort aufbauende Druck kann auf einfache Weise durch die Drehzahl eingestellt werden. Eine druckgeregelte Schlauchrollenpumpe bedarf mindestens eines Drucksensors mit Regelkreis; eine Schlauchrollenpumpe mit

Überdruckbypassventil muss exakt auf den erlaubten Druck kalibriert werden. Durch Verwenden von Zentrifugalpumpen ist die erfindungsgemäße Bilanziereinrichtung in bestimmten Ausführungsformen somit weniger aufwändig.

Im Folgenden wird die vorliegende Erfindung beispielhaft unter Bezugnahme auf die beigefügte Zeichnung beschrieben. In der Zeichnung bezeichnen gleiche Bezugszeichen gleiche oder identische Bauteile bzw. Komponenten. Es gilt:
- Fig. 1: zeigt schematisch vereinfacht eine erfindungsgemäße Bilanziereinrichtung während eines ersten Arbeitstaktes;
- Fig. 2: zeigt als Diagramm einen Druckverlauf in Abhängigkeit der Zeit während einer Bilanzierkammerfüllung;
- Fig. 3: zeigt als Diagramm eine Druckdifferenz zwischen dem Pumpenaus- und dem Pumpeneinlass einer Zentrifugalpumpe in Abhängigkeit vom Volumenstrom;
- Fig. 4: zeigt schematisch vereinfacht die erfindungsgemäße Bilanziereinrichtung der Fig. 1 während eines zweiten Arbeitstaktes;
- Fig. 5: zeigt schematisch vereinfacht die erfindungsgemäße Bilanziereinrichtung der Fig. 1 mit zwei weiteren, der Bilanzierkammer nachgeschalteten Zentrifugalpumpen;
- Fig. 6: zeigt schematisch vereinfacht die erfindungsgemäße Bilanziereinrichtung mit der Bilanzierkammer, Ventilen, Zentrifugalpumpen, wobei eine der nachgeschalteten Zentrifugalpumpen in einer anderen Richtung dreht;
- Fig. 7: zeigt schematisch vereinfacht eine Zentrifugalpumpe mit magnetischer Lagerung und magnetischem Antrieb;
- Fig. 8: zeigt schematisch vereinfacht eine erfindungsgemäße Behandlungsvorrichtung mit Bilanziereinrichtung und einer externer medizinischer Funktionseinrichtung; und
- Fig. 9: zeigt schematisch vereinfacht eine erfindungsgemäße Bilanziereinrichtung einer weiteren Ausführungsform während eines ersten Arbeitstaktes.

Die Bilanziereinrichtung wird im Folgenden beispielhaft als Teil einer Blutbehandlungsvorrichtung zur Dialyse beschrieben. Es ist vorgesehen, die einem Patienten zu- und abgeführte Dialysierflüssigkeit zu bilanzieren. Prinzipiell kann jedoch auch vorgesehen sein, das Blut des Patienten zu bilanzieren.

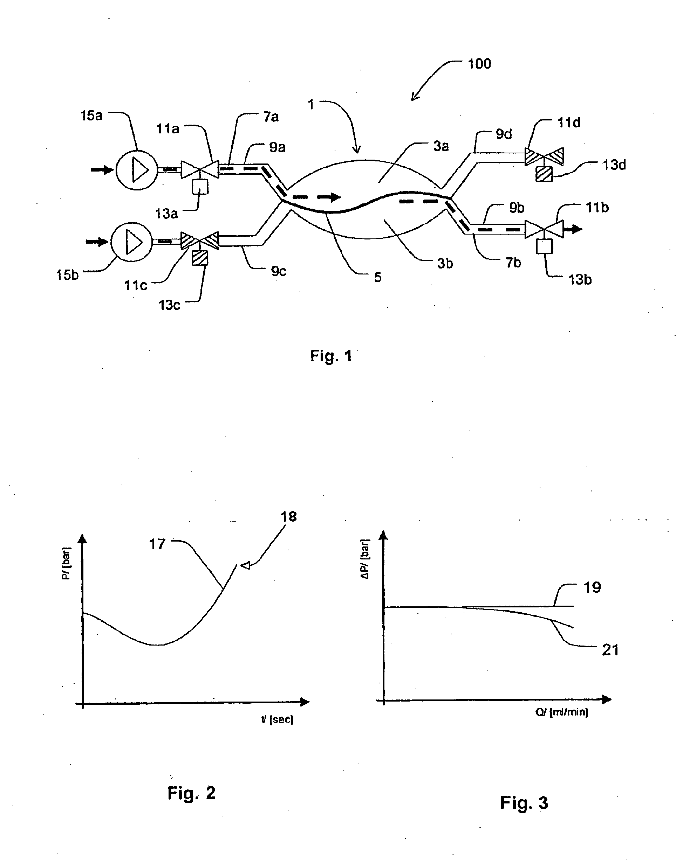

**Fig. 1** zeigt eine Bilanziereinrichtung 100 gemäß der vorliegenden Erfindung mit einer Bilanzierkammer 1.

Wie in Fig. 1 gezeigt, ist die Bilanzierkammer 1 in eine erste Bilanzierkammerhälfte 3a und eine zweite Bilanzierkammerhälfte 3b unterteilt. Prinzipiell muss die Bilanzierkammer jedoch nicht in zwei im Wesentlichen oder vollständig gleich große Bilanzierkammerhälften aufgeteilt sein.

Die erste Bilanzierkammerhälfte 3a ist durch eine fluidundurchlässige Membran 5 von der zweiten Bilanzierkammerhälfte 3b getrennt.

Die erste Bilanzierkammerhälfte 3a wird mit einem Strom 7a einer Dialysierflüssigkeit über einen Schlauch 9a befüllt. Ein Ventil 11a befindet sich dabei in geöffneter Position mittels eines Stellmechanismus 13a.

Die Fördereinrichtung kann eine Zentrifugalpumpe sein. Wie in Fig. 1 gezeigt, wird die erste Kammerhälfte 3a mittels einer Zentrifugalpumpe 15a befüllt.

Das Ventil 11a kann als Schlauchklemme (oder allgemein als Quetschmechanismus) ausgeführt sein. Eine solche Schlauchklemme kann durch einen elektrisch angesteuerten Aktuator geöffnet und geschlossen werden. Dies hat den Vorteil, dass das medizinische Fluid im Wesentlichen nur Kontakt mit dem Schlauch 9a, nicht jedoch mit Teilen des Ventils 11a oder dem Stellmechanismus 13a hat. Dies kann vorteilhaft dazu beitragen, eine Kontaminationsgefahr für die medizinischen Fluide zu verringern.

Ein zweiter Strom 7b der Dialysierflüssigkeit wird durch einen Schlauch 9b aus der zweiten Bilanzierkammerhälfte 3b abgeführt. Ein Ventil 11b befindet sich dabei ebenfalls in geöffneter Position, vermittelt durch einen Stellmechanismus 13b.

Die zweite Bilanzierkammerhälfte 3b kann entleert werden. Das Austragen oder Abführen von Dialysierflüssigkeit aus der zweiten Bilanzierkammerhälfte 3b kann gleichzeitig mit dem Einbringen oder Eintragen von Dialysierflüssigkeit in die erste Bilanzierkammerhälfte 3a erfolgen.

Ventile 11c und 11d sind, wie in Fig. 1 gezeigt, jeweils mittels der entsprechenden Stellmechanismen 13c und 13d geschlossen. In Schläuchen 9c und 9d wird kein Fluid gefördert.

**Fig. 2** zeigt ein Diagramm mit einem Druckverlauf 17 während einer Bilanzierkammerfüllung in Abhängigkeit von der Zeit.

Ein Anfangsdruck bei t=0 entspricht einem Druck, mit welchem in Fig. 1, auf welche im Folgenden ebenfalls Bezug genommen wird, der Strom 7a der Dialysierflüssigkeit durch den Schlauch 9a nach Öffnen des Ventils 11a in die erste Bilanzierkammerhälfte 3a eingebracht wird. Damit der Strom 7b an Dialysierflüssigkeit durch den Schlauch 9b aus der zweiten Bilanzierkammerhälfte 3b abfließen kann, sollte das Ventil 11b geöffnet sein.

Während die erste Bilanzierkammerhälfte 3a gefüllt und die zweite Bilanzierkammerhälfte 3b entleert wird, sinkt der Druck in der Bilanzierkammer 1 zunächst ab.

Wenn die erste Bilanzierkammerhälfte 3a gefüllt ist, steigt der Druck an. Ein Enddruck 18, der dem Endpunkt des Druckverlaufs bei Bilanzkammerfüllung 17 und damit dem maximalen Fülldruck entspricht, kann von dem Druck abhängen, den die Zentrifugalpumpe 15a aufbringt. Dieser Druck kann wiederum von mehreren Parametern der Zentrifugalpumpe abhängen, beispielsweise dem Bauprinzip der Zentrifugalpumpe (Radial-, Axial-, Diagonalpumpe, Laufradform, Durchmesser des Laufrads usw.) und/oder der eingestellten Drehzahl der Zentrifugalpumpe 15a und damit dem eingestellten Arbeitspunkt. Weiterhin kann der Enddruck von der Vorlast der Zentrifugalpumpe 15a abhängen, d. h., dem Druck, der an einem Dialysateinlass der Zentrifugalpumpe 15a anliegt.

**Fig. 3** zeigt ein Diagramm mit einer Druckdifferenz ΔP zwischen dem Pumpenaus- und dem Pumpeneinlass einer Zentrifugalpumpe 15a (Ordinate) in Abhängigkeit vom Volumenstrom Q der medizinischen Fluide(Abszisse).

Bei einer Kennlinie 19 einer idealen Druckquelle, welche zum Vergleich angegeben ist, ist die Druckdifferenz ΔP unabhängig vom Volumenstrom Q. Die Höhe bzw. das Ausmaß der Druckdifferenz ΔP hängt unter anderem von der eingestellten Drehzahl einer Zentrifugalpumpe ab.

Die tatsächlichen Druckverläufe (ΔP, Q) weichen i.d.R. von der idealen Kennlinie ab. Einen möglichen Druckverlauf einer Kennlinie für eine druckbegrenzte Fördereinrichtung wie die Zentrifugalpumpe 15a der erfindungsgemäßen Bilanziereinrichtung 100 der Fig. 1 zeigt die Kennlinie 21 einer Zentrifugalpumpe. Man erkennt, dass mittels der Zentrifugalpumpe 15a eine gute Näherung des Druckverlaufs an die ideale Kennlinie erzielbar ist. Fig. 3 zeigt auch, dass die Zentrifugalpumpe 15a als druckbegrenzte Fördereinrichtung im Sinne der vorliegenden Erfindung verstanden werden kann: der Pumpenauslass-Druck steigt ab Erreichen eines bestimmten Drucks trotz zunehmenden Volumenstroms nicht weiter an.

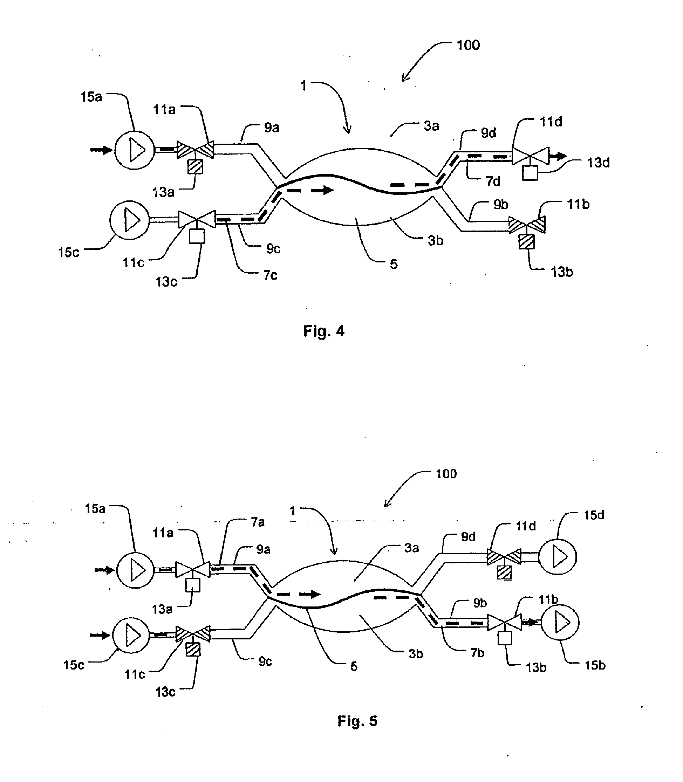

**Fig. 4** zeigt die Bilanziereinrichtung 100 der Fig. 1 während eines zweiten Arbeitstaktes. Der zweite Arbeitstakt kann dem ersten Arbeitstakt entsprechend Fig. 1 nachfolgen.

Ein Strom 7c an Dialysierflüssigkeit wird im zweiten Arbeitstakt von einer Zentrifugalpumpe 15c durch den Schlauch 9c in die zweite Kammerhälfte 3b gefördert. Gleichzeitig wird ein Strom 7d an Dialysierflüssigkeit aus der ersten Kammerhälfte 3a entleert.

**Fig. 5** zeigt die Bilanziereinrichtung 100 der Fig. 1 mit zwei zusätzlichen, der Bilanzierkammer 1 nachgeschalteten Zentrifugalpumpen 15b und 15d.

Die in der erfindungsgemäßen Bilanziereinrichtung 100 der Fig. 5 angeordneten Zentrifugalpumpen 15a-d fördern alle in die gleiche Förderrichtung, wie mit den nach links (bezogen auf die Darstellung der Fig. 5) zeigenden Pumpenpfeilköpfen angedeutet ist.

Mit Hilfe der nachgeschalteten Zentrifugalpumpen 15b und 15d kann die Entleerung der beiden Kammerhälften 3a und 3b unterstützt werden. Dies kann vorteilhaft sein, um beispielsweise einen maximalen Druck (siehe in Fig. 2 den Endpunkt 18 der Kurve des Druckverlaufs) in der Bilanzierkammer 1 zu verringern oder niedrig zu halten. Niedrige Drücke in der Bilanzierkammer 1 können wiederum vorteilhaft dazu beitragen, die Konstruktion (wie z.B. eine geringere Steifigkeit, geringere Materialstärken etc.) der Bilanziereinrichtung 100 wie oben ausgeführt zu vereinfachen. Letzteres kann insbesondere bei einer Ausgestaltung der Bilanziereinrichtung 100 als Teil eines Disposables vorteilhaft sein.

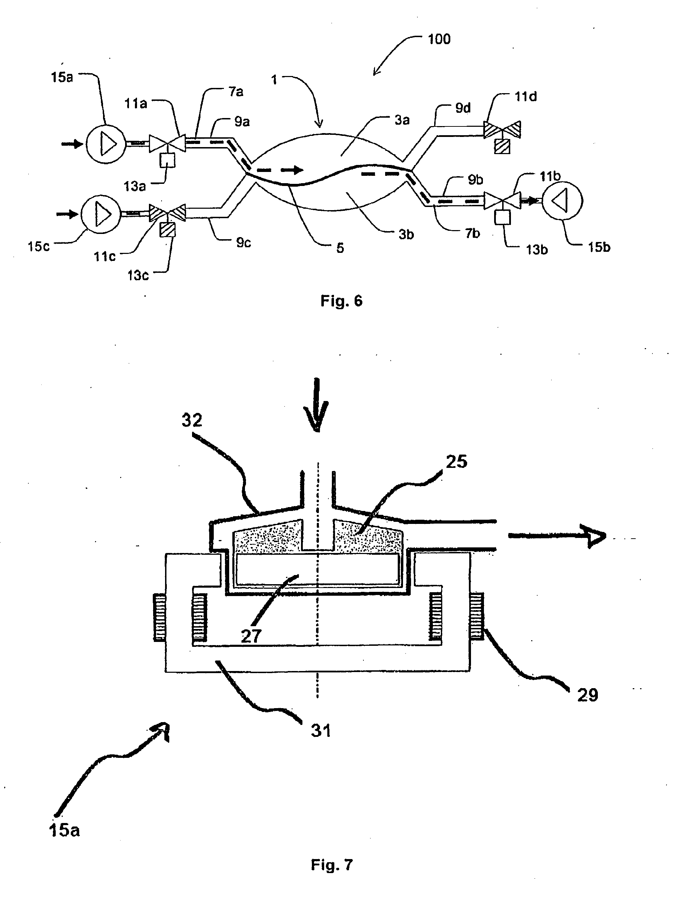

**Fig. 6** zeigt die Bilanziereinrichtung 100 mit der Bilanzierkammer 1 ähnlich wie in Fig. 5, mit dem Unterschied, dass die Zentrifugalpumpe 15b vorgesehen und konfiguriert ist, auch in eine andere Richtung zu laufen bzw. in entgegengesetzte Richtungen zu fördern, wie mit dem nach links (bezogen auf die Darstellung der Fig. 6) zeigenden Pumpenpfeilkopf angedeutet ist.

Die Zentrifugalpumpe 15b arbeitet, wenn sie in entgegengesetzter Drehrichtung läuft, als Druckminderer, insbesondere als einstellbarer Druckminderer.

In der Ausführungsform der Fig. 6 können Einlass und Auslass der Zentrifugalpumpe 15b vertauscht sein.

Ein "Vertauschen" von Einlass und Auslass kann auf verschiedene Weise erfolgen. Beispiele hierfür sind ein umgekehrtes Einbauen der Zentrifugalpumpe, ein Vorsehen von entsprechend angeordneten und angesteuerten Ventilen und dergleichen.

Bei entsprechend angeordneten und angesteuerten Ventilen können diese bevorzugt durch Aktoren einer Dialysemaschine über eine flexible Membran betätigt werden, z.B. durch Abdrücken und/oder Freigeben der relevanten Fluidwege.

Die Richtungsumkehr kann ergänzend oder alternativ vorgesehen sein. Die betrachtete Fördereinrichtung kann vorgesehen und konfiguriert sein, in einer oder in zwei, einander entgegengesetzten Richtungen betrieben zu werden.

**Fig. 7** zeigt eine Zentrifugalpumpe 15a mit einem Laufrad 25 als Rotationsabschnitt, einem Rotor 27, Spulen 29 und einem Stator 31. Die Zentrifugalpumpe 15a weist ein Gehäuse 32 auf mit einem Zulass und einem Auslass (durch Pfeile in Fig. 7 als solche erkennbar).

Die Zentrifugalpumpe 15a wird in der gezeigten Strömungsrichtung durchströmt. Der Antrieb des Laufrades 25 erfolgt durch ein umlaufendes elektromagnetisches Feld, erzeugt mittels Ansteuern der Spulen 29 des Stators 31.

In das Laufrad 25 können Laufradmagnete oder wenigstens ferromagnetische Materialien integriert sein.

Die Lagerung des Laufrades 25 kann dann einerseits mittels der Laufradmagnete und andererseits mittels außerhalb der Zentrifugalpumpe vorgesehener Magnete erfolgen. Die Magnete können umlaufend in der gleichen Drehbewegung wie das Laufrad 25 angeordnet sein. Anstatt der umlaufenden Magnete oder ergänzend hierzu kann auch ein umlaufendes elektromagnetisches Feld in einer Spulenanordnung das Laufrad 25 lagern bzw. in einer stabilen umlaufenden Position fixieren. Diese Ausführungsform ist - obgleich nicht in den Figuren dargestellt - ebenfalls von der Erfindung umfasst.

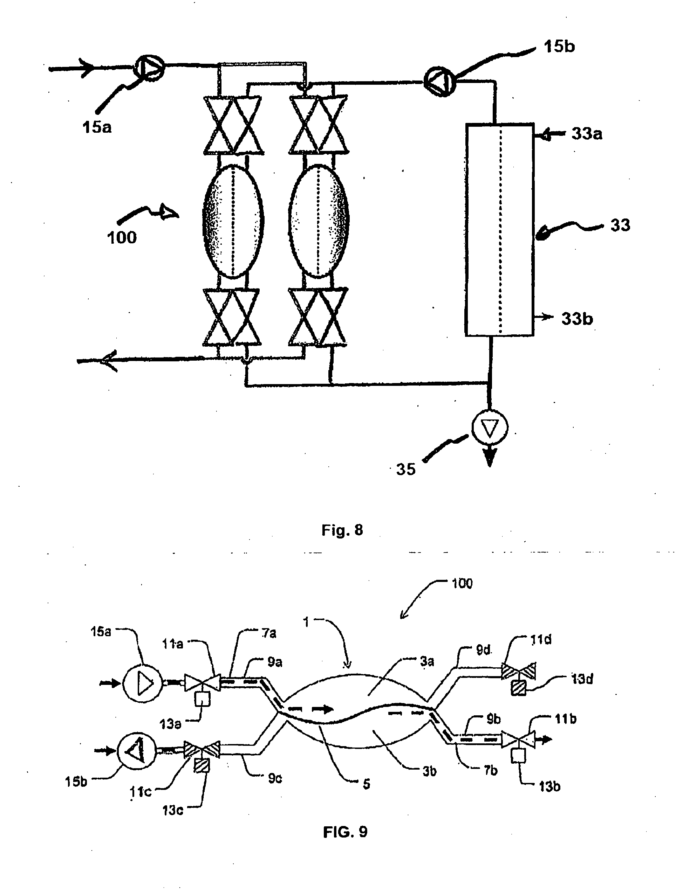

**Fig. 8** zeigt schematisch vereinfacht eine erfindungsgemäße Bilanziereinrichtung 100 und eine erfindungsgemäße Behandlungsvorrichtung 300 mit einem Dialysator 33 mit einem Bluteinlass 33a und einem Blutauslass 33b, sowie weiteren Elementen.

**Fig. 9** zeigt in Anlehnung an die Fig. 1 schematisch vereinfacht eine erfindungsgemäße Bilanziereinrichtung einer weiteren Ausführungsform während eines ersten Arbeitstaktes. Zu erkennen ist, dass die Zentrifugalpumpe 15b in entgegengesetzter Richtung zur Förderrichtung der Zentrifugalpumpe 15a fördert. Mittels dieser Ausführung der in entgegengesetzte Richtungen pumpenden Fördereinrichtungen kann einem ggf. zu hohen Eingangsdruck vorteilhaft entgegen gewirkt werden oder dieser gemindert werden. Dies kann der Fall sein, wenn das Dialysat aus einem RO-Wasser (reverse osmosis water) und Konzentraten hergestellt ist. Dabei kann der RO-Wasseranschluss einen derart hohen Leitungsdruck aufweisen, dass die Bilanziereinrichtung durch diesen beschädigt werden könnte.

## Patentansprüche

1. Bilanziereinrichtung (100) für medizinische Fluide mit
- wenigstens einer Bilanzierkammer (1) und
- wenigstens einer Fördereinrichtung zum Befüllen der Bilanzierkammer (1),
wobei die Fördereinrichtung eine druckbegrenzte Fördereinrichtung ist,
**dadurch gekennzeichnet, dass** die Fördereinrichtung eine Zentrifugalpumpe ist, welche aufgrund ihrer Bauart keinen Druck über einen vorbestimmten Druck hinaus aufbaut, ohne hierbei durch weitere Elemente unterstützt zu sein.

2. Bilanziereinrichtung (100) nach Anspruch 1, wobei die Fördereinrichtung wenigstens einen Rotationsabschnitt aufweist, welcher vorgesehen und ausgestaltet ist, um mittels eines externen Antriebs magnetisch oder mittels eines elektrischen Feldes antreibbar zu sein.

3. Bilanziereinrichtung (100) nach Anspruch 2, wobei der externe Antrieb nicht Teil der Bilanzierungseinrichtung ist.

4. Bilanziereinrichtung (100) nach einem der vorangegangenen Ansprüche, wobei das medizinische Fluid ausgewählt ist aus Blut, Dialysierflüssigkeit, Substituatflüssigkeit, Medikamenten, Medikamentenzubereitungen sowie Mischungen oder Kombinationen derselben.

5. Bilanziereinrichtung (100) nach einem der vorangegangenen Ansprüche, wobei die Fördereinrichtung wenigstens eine erste Fördereinrichtung aufweist, um in einer ersten Richtung zu fördern, und wenigstens eine zweite Fördereinrichtung aufweist, um in einer zweiten, der ersten entgegengesetzten Richtung, zu fördern.

6. Bilanziereinrichtung (100) nach einem der vorangegangenen Ansprüche, wobei die Fördereinrichtung wenigstens eine erste Fördereinrichtung aufweist, um in einer ersten Richtung zu fördern, und wenigstens eine zweite Fördereinrichtung aufweist, ebenfalls in der ersten Richtung zu fördern.

7. Externe medizinische Funktionseinrichtung (200) mit wenigstens einer Bilanziereinrichtung (100) gemäß einem der Ansprüche 1 bis 6.

8. Externe medizinische Funktionseinrichtung (200) nach Anspruch 7, ausgestaltet als externer oder extrakorporaler Blutkreislauf oder Blutkassette.

9. Externe medizinische Funktionseinrichtung (200) nach Anspruch 7 oder 8, ausgestaltet als Disposable oder Einwegartikel zum Einlegen in eine Blutbehandlungsvorrichtung, wobei die Funktionseinrichtung (200) wenigstens eine druckbegrenzte Fördereinrichtung, und wenigstens eine integrierte Bilanzierkammer, welche mittels einer oder wenigstens einer flexiblen Membran in zwei Kammerabschnitte untereilt ist, aufweist.

10. Behandlungsvorrichtung (300) zum Behandeln medizinischer Fluide, welche wenigstens eine Bilanziereinrichtung (100) gemäß einem der Ansprüche 1 bis 6 aufweist.

11. Behandlungsvorrichtung (300) nach Anspruch 10, welche funktionell mit einer Bilanziereinrichtung (100) gemäß einem der Ansprüche 1 bis 6 und/oder mit einer externen medizinischen Funktionseinrichtung (200) gemäß Anspruch 7 bis 9 verbunden ist.

12. Behandlungsvorrichtung (300) nach einem der Ansprüche 10 bis 11, welche als Blutbehandlungsvorrichtung, insbesondere als Hämodialysemaschine, ausgestaltet ist.

## Claims

1. A balancing unit (100) for medical fluids comprising
- at least one balancing chamber (1) and
- at least one conveying means for filling the balancing chamber (1),
wherein the conveying means is a pressure limited conveying means,
**characterized in that** the conveying means is a centrifugal pump which, due to its type, does not build up a pressure above a predetermined pressure, without being supported by further elements.

2. The balancing unit (100) according to claim 1, wherein the conveying means comprises at least one rotational section provided and designed for being actuated magnetically by means of an external actuator or by means of an electrical field.

3. The balancing unit (100) according to claim 2, wherein the external actuator is not part of the balancing unit.

4. The balancing unit (100) according to anyone of the preceding claims, wherein the medical fluid is selected from blood, dialysis liquid, substituate liquid, drugs, drug preparations as well as mixtures or combinations thereof.

5. The balancing unit (100) according to anyone of the preceding claims, wherein the conveying means comprises at least one first conveying means provided to convey in a first direction and at least one second conveying means provided to convey in a second direction opposite to the first direction.

6. The balancing unit (100) according to anyone of the preceding claims, wherein the conveying means comprises at least one first conveying means provided to convey in a first direction and at least one second conveying means provided to convey in the first direction, too.

7. An external medical functional means (200) comprising at least one balancing unit (100) according to anyone of claims 1 to 6.

8. The external medical functional means (200) according to claim 7, configured as an external or extracorporeal blood circuit or blood cassette.

9. The external medical functional means (200) according to claim 7 or 8, configured as a disposable or one-way article for insertion in a blood treatment apparatus, wherein the functional means (200) comprises at least one pressure limited conveying means and at least one integrated balancing chamber which is separated into two chamber sections by means of one or at least one flexible membrane.

10. A treatment apparatus (300) for treating medical fluids comprising at least one balancing unit (100) according to anyone of claims 1 to 6.

11. The treatment apparatus (300) according to claim 10, functionally connected with a balancing unit (100) according to anyone of claims 1 to 6 and/or with an external medical functional means (200) according to claims 7 to 9.

12. The treatment apparatus (300) according to anyone of claims 10 to 11, configured as a blood treatment apparatus, in particular as a hemodialysis machine.

## Revendications

1. Equipement d'équilibrage (100) pour des fluides médicaux comprenant
- au moins une chambre d'équilibrage (1) und
- au moins un équipement de convoyage pour remplir la chambre d'équilibrage (1),
où l'équipement de convoyage est un équipement de convoyage à pression limitée,
**caractérisé en ce que** l'équipement de convoyage est une pompe centrifuge qui, en raison de son type de construction, ne peut établir une pression dépassant une pression supérieure à une pression prédéterminée sans être soutenue par d'autres éléments.

2. Equipement d'équilibrage (100) selon la première revendication, où l'équipement de convoyage présente au moins une partie rotative, laquelle est prévue et configurée pour être entrainée au moyen d'un mécanisme d'entraînement externe de façon magnétique ou au moyen d'un champ électrique.

3. Equipement d'équilibrage (100) selon la seconde revendication, où le mécanisme d'entraînement externe ne fait pas partie de l'équipement d'équilibrage.

4. Equipement d'équilibrage (100) selon l'une des revendications précédentes, où le fluide médical est sélectionné parmi un groupe comprenant le sang, un liquide de dialyse, un liquide de substitution, des médicaments, des préparations de médicaments ainsi que des mélanges ou des combinations de ceux-ci.

5. Equipement d'équilibrage (100) selon l'une des revendications précédentes, où l'équipement de convoyage présente au moins un premier équipement de convoyage pour faire avancer dans une première direction et au moins un deuxième équipement de convoyage pour faire avancer dans une deuxième direction, la deuxième direction étant opposée à la première direction.

6. Equipement d'équilibrage (100) selon l'une des revendications précédentes, où l'équipement de convoyage présente au moins un premier équipement de convoyage pour faire avancer dans une première direction et au moins un deuxième équipement de convoyage pour faire également avancer dans la première direction.

7. Dispositif médical fonctionnel externe (200) comprenant au moins un équipement d'équilibrage (100) selon l'une des revendications 1 à 6.

8. Dispositif médical fonctionnel externe (200) selon la revendication 7, configuré en tant que circuit externe ou extracorporal de circulation de sang ou en tant que cassette à sang.

9. Dispositif médical fonctionnel externe (200) selon la revendication 6 ou 7, configuré comme article à usage unique ou jetable, prévu pour être inséré dans un appareillage de traitement du sang, où le dispositif fonctionnel (200) présente au moins un équipement de convoyage à pression limitée et au moins chambre d'équilibrage intégrée, laquelle est subdivisée en deux sections au moyen d'une ou d'au moins une menbrane flexible.

10. Appareillage de traitement (300) pour traiter des fluides médicaux, lequel présente au moins un équipement d'équilibrage (100) selon l'une des revendications 1 à 6.

11. Appareillage de traitement (300) selon la revendication 10, lequel est relié de façon fonctionnelle avec un équipement d'équilibrage (100) selon l'une des revendications 1 à 6 et/ou avec dispositif fonctionnel médical externe (200) selon l'une des revendications 7 à 9.

12. Appareillage de traitement (300) selon l'une des revendications 10 à 11, lequel est configuré comme un appareillage de traitement du sang, en particulier comme machine d'hémodialyse.
